# EUROPEAN PATENT APPLICATION

(11) **EP 4 574 070 A1**
(43) Date of publication of application: **25.06.2025**
(21) Application number: 24220422.0
(22) Date of filing: 17.12.2024
(51) Int. Cl.: A61B 18/14, A61B 5/287, A61B 5/00, A61B 18/00

(54) **MEDICAL PROBE WITH SPINES FOR PULMONARY VEIN ISOLATION**

(30) Priority: 18.12.2023 US 202318544155
(71) Applicant: Biosense Webster (Israel) Ltd., 2066717 Yokneam (IL)
(72) Inventor: GARCIA, Roy, Irvine, 92618 (US); DATTA, Keshava, Irvine, 92618 (US); PHAM, Cuong, Irvine, 92618 (US); BAH, Abubakarr, Irvine, 92618 (US); PADILLA, Ricardo, Irvine, 92618 (US); BLUMENSTYK, David, Irvine, 92618 (US); TROUTMAN, Terrence, Irvine, 92618 (US)
(74) Representative: Carpmaels & Ransford LLP

(57) **Abstract**

The disclosed technology includes a distal tip of a medical probe. The distal tip includes a neck and spines that extend along a longitudinal axis. The neck extends from a proximal end of the distal tip. The spines are connected to the neck and extending from an intermediate section of the distal tip to a distal end of the distal tip. The spines are movable between a collapsed configuration and an expanded configuration. In the collapsed configuration, the spines extend along the longitudinal axis. In the expanded configuration, each spine includes a curved section that bends away from the longitudinal axis and a planar section extending from the curved section to the distal end. Further, in the expanded configuration, each planar section extending along a plane that is approximately orthogonal to the longitudinal axis.

## Description

### FIELD

The present technology relates generally to medical devices, and in particular medical probes with electrodes, and further relates to, but not exclusively, medical probes suitable for use to map and/or ablate tissue.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

In current practice, the effectiveness of the delivery of IRE energy is dependent on the skill of the physician, meaning the patient can suffer from incomplete isolation of target areas, such as the pulmonary vein. In order to effectively deliver the IRE energy to perform pulmonary vein isolation (PVI), ablation catheters typically need to be reoriented multiple times during a procedure, which increases procedure time as well as complicates the ablation process. Accordingly, there is a need for an improved end effector of a medical probe that addresses these problems.

### SUMMARY

There is provided, in accordance with the disclosed technology, a distal tip of a medical probe. The distal tip comprises a spine framework having a proximal end, an intermediate section, and a distal end and extending along a longitudinal axis. The spine framework comprises a neck and a plurality of spines. The neck extends from the proximal end. The spines are connected to the neck and extend along the intermediate section to the distal end. The spines are movable between (1) a collapsed configuration, where the spines extend along the longitudinal axis and (2) an expanded configuration, where each spine comprises a curved section that bends away from the longitudinal axis and a planar section extending from the curved section to the distal end, each planar section extending along a plane that is approximately orthogonal to the longitudinal axis.

There is further provided, in accordance with the disclosed technology, a distal tip of a medical probe. The distal tip comprises a spine framework having a proximal end, an intermediate section, and a distal end and extending along a longitudinal axis. The spine framework comprises a neck, a plurality of spines, and a crown. The neck extends from the proximal end. The spines extend along the intermediate section. Each spine comprises (1) a first straight section connected to the neck and (2) a second straight section comprising a first end and a second end. The first straight section is connected to the second straight section at a joint intermediate the first end and the second end. The crown is connected to the second straight section and extends to the distal end. The spine framework is moveable between (1) a collapsed configuration, where each connected first straight section and second straight section are oriented at a first angle relative to one another, and (2) an expanded configuration, where each connected first straight section and second straight section are oriented at a second angle relative to one another, the second angle being smaller than the first angle.

There is further provided, in accordance with the disclosed technology, a system that includes a medical probe, a guidewire, and a guiding sheath. The medical probe comprises an elongated shaft and a distal tip. The elongated shaft extends along a longitudinal axis. The distal tip comprises a spine framework having a proximal end, an intermediate section, and a distal end and extending along a longitudinal axis. The spine framework comprises a neck and a plurality of spines. The neck extends from the proximal end. The spines are connected to the neck and extend along the intermediate section to the distal end. The spines are movable between (1) a collapsed configuration, where the spines extend along the longitudinal axis, and (2) an expanded configuration, where each spine comprises a curved section that bends away from the longitudinal axis and a planar section extending from the curved section to the distal end, each planar section extending along a plane that is approximately orthogonal to the longitudinal axis. The guidewire extends through the elongated shaft and the distal tip. The guiding sheath is slidable relative to the distal tip to cause the spines to move between the collapsed configuration and the expanded configuration.

There is further provided, in accordance with the disclosed technology, a system that includes a medical probe, a guidewire, and a rod or wire. The medical probe comprises and elongated shaft and a distal tip. The elongated shaft extends along a longitudinal axis. The distal tip comprises a spine framework having a proximal end, an intermediate section, and a distal end and extending along a longitudinal axis. The spine framework comprises a neck, a plurality of spines, a first straight section, a second straight section, and a crown. The neck extends from the proximal end. The spines extend along the intermediate section. Each spine comprises (1) a first straight section connected to the neck and (2) a second straight section comprising a first end and a second end, the first straight section being connected to the second straight section at a joint intermediate the first end and the second end. The crown is connected to the second straight section and extending to the distal end. The spine framework is moveable between (1) a collapsed configuration, where each connected first straight section and second straight section are oriented at a first angle relative to one another and (2) an expanded configuration, where each connected first straight section and second straight section are oriented at a second angle relative to one another, the second angle being smaller than the first angle. The guidewire extends through the elongated shaft and the distal tip. The rod or wire is connected to one of the neck or the crown and operable to move the spine framework between the collapsed configuration and the expanded configuration.

There is further provided, in accordance with the disclosed technology, a method of making a distal dip of a medical probe. The method comprises the step of cutting a tube, that extends along a longitudinal axis, to define a neck and a plurality of spines. The method comprises the step of shape setting the spines such that each spine comprises a curved section that bends away the longitudinal axis and a planar section extending from the curved section, each planar section extending along a plane that is approximately orthogonal to the longitudinal axis.

There is further provided, in accordance with the disclosed technology, a method of making a distal dip of a medical probe. The method comprises the step of shape setting a plurality of rods such that each rod comprises (1) a first end extending along a longitudinal axis, (2) a first curved section extending from the first end and bending away from the longitudinal axis, (3) a planar section extending from the curved section that forms a partial loop shape, the planar section extending along a plane that is approximately orthogonal to the longitudinal axis; (4) a second curved section extending from the planar section and bending towards the longitudinal axis, and (5) a second end extending from the second curved section and extending along the longitudinal axis. The method comprises the step of connecting the rods together such that the first ends and the second ends define a lumen.

There is further provided, in accordance with the disclosed technology, a method of using a medical probe. The medical probe comprises a distal tip that comprises a spine framework having a proximal end, an intermediate section, and a distal end and extending along a longitudinal axis. The spine framework comprises a neck extending from the proximal end and a plurality of spines connected to the neck and extending along the intermediate section to the distal end. A plurality of electrodes are connected to the spine framework. The method comprises the step of moving the spines from (1) a collapsed configuration, where the spines extend along the longitudinal axis, to (2) an expanded configuration, where each spine comprises a curved section that bends away the longitudinal axis and a planar section extending from the curved section to the distal end, each planar section extending along a plane that is approximately orthogonal to the longitudinal axis. The method comprises the step of positioning the planar sections against a pulmonary vein opening such that the electrodes are placed in contact with the pulmonary vein opening.

There is further provided, in accordance with the disclosed technology, a method of using a medical probe. The medical probe comprises a distal tip that comprises a spine framework having a proximal end, an intermediate section, and a distal end and extending along a longitudinal axis. The spine framework comprises a neck extending from the proximal end, a plurality of spines extending along the intermediate section, and a crown connected to the second straight section and extending to the distal end. Each spine comprises a first straight section connected to the neck and a second straight section comprising a first end and a second end. The first straight section is connected to the second straight section at a joint intermediate the first end and the second end. The method comprises the step of moving spine framework from (1) a collapsed configuration, where each connected first straight section and second straight section are oriented at a first angle relative to one another, to (2) an expanded configuration, where each connected first straight section and second straight section are oriented at a second angle relative to one another, the second angle being smaller than the first angle. The method comprises the step of positioning the second straight sections against a pulmonary vein opening such that the electrodes are placed in contact with the pulmonary vein opening.

### BRIEF DESCRIPTION OF THE DRAWINGS

FIG. 1 is a schematic pictorial illustration of a medical system including a medical device that includes a medical probe with a distal tip with electrodes, in accordance with the disclosed technology;
FIG. 2A is a schematic pictorial illustration showing a perspective view of a spine framework of a distal tip of the medical probe in a collapsed configuration, in accordance with the disclosed technology;
FIG. 2B is a schematic pictorial illustration showing a perspective view of the spine framework of FIG. 2A in an expanded configuration, in accordance with the disclosed technology;
FIG. 2C is a schematic pictorial illustration showing a perspective view of a distal side of the distal tip, with the spine framework of FIG. 2B, in the expanded configuration, in accordance with the disclosed technology;
FIG. 2D is a schematic pictorial illustration showing a perspective view of a proximal side of the distal tip of FIG. 2C in the expanded configuration, in accordance with the disclosed technology;
FIG. 3 is a schematic pictorial illustration showing a sectional view of the distal tip of FIG. 2C in the collapsed configuration and proximal an ostium of a pulmonary vein, in accordance with the disclosed technology;
FIG. 4 is a schematic pictorial illustration showing a sectional view of the distal tip of FIG. 2C in the expanded configuration and pressed against the ostium of the pulmonary vein, in accordance with the disclosed technology;
FIG. 5A is a schematic pictorial illustration showing a perspective view of another spine framework of another distal tip of the medical probe in the expanded configuration, in accordance with the disclosed technology;
FIG. 5B is a schematic pictorial illustration showing a perspective view of a proximal side of the another distal tip, with the spine framework of FIG. 5A, of the medical probe in the expanded configuration, in accordance with the disclosed technology;
FIG. 6 is a schematic pictorial illustration showing a sectional view of the distal tip of FIG. 5B in the collapsed configuration and proximal the ostium of the pulmonary vein, in accordance with the disclosed technology;
FIG. 7 is a schematic pictorial illustration showing a sectional view of the distal tip of FIG. 5B in the expanded configuration and pressed against the ostium of the pulmonary vein, in accordance with the disclosed technology;
FIG. 8A is a schematic pictorial illustration showing a perspective view of yet another spine framework of yet another distal tip of the medical probe in the expanded configuration, in accordance with the disclosed technology;
FIG. 8B is a schematic pictorial illustration showing a perspective view of a proximal side of the yet another distal tip, with the spine framework of FIG. 8A, of the medical probe in the expanded configuration, in accordance with the disclosed technology;
FIG. 9 is a schematic pictorial illustration showing a sectional view of the distal tip of FIG. 8B in the collapsed configuration and proximal the ostium of the pulmonary vein, in accordance with the disclosed technology;
FIG. 10 is a schematic pictorial illustration showing a sectional view of the distal tip of FIG. 8B in the expanded configuration and pressed against the ostium of the pulmonary vein, in accordance with the disclosed technology;
FIG. 11A is a schematic pictorial illustration showing a perspective view of yet another spine framework of yet another distal tip of the medical probe in the expanded configuration, in accordance with the disclosed technology;
FIG. 11B is a schematic pictorial illustration showing a perspective view of the spine framework of FIG. 11A in the collapsed configuration, in accordance with the disclosed technology;
FIG. 11C is a schematic pictorial illustration showing a perspective view of a distal side of the yet another distal tip, with the spine framework of FIG. 11A, of the medical probe in the expanded configuration, in accordance with the disclosed technology;
FIG. 11D is a schematic pictorial illustration showing a perspective view of a proximal side of the distal tip of FIG. 11C in the expanded configuration, in accordance with the disclosed technology;
FIG. 12 is a schematic pictorial illustration showing a sectional view of the distal tip of FIG. 11C in the collapsed configuration and proximal the ostium of the pulmonary vein, in accordance with the disclosed technology; and
FIG. 13 is a schematic pictorial illustration showing a sectional view of the distal tip of FIG. 11C in the expanded configuration and pressed against the ostium of the pulmonary vein, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

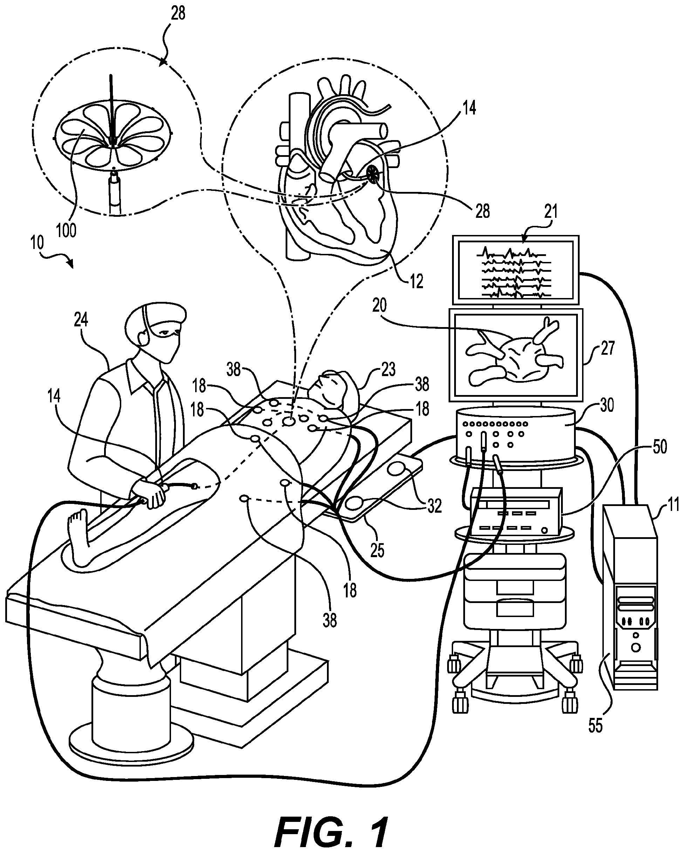

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location, such as the ostium 34 of the pulmonary vein (PV). The plurality of catheters may include catheters dedicated for sensing Intracardiac Electrogram (IEGM) signals, catheters dedicated for ablating and/or catheters dedicated for both sensing and ablating. An example medical device/probe, e.g., a catheter 14 (also referred to synonymously herein as a probe 14), that is configured for sensing IEGM is illustrated herein. Physician 24 brings a distal tip of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 configured to sense the IEGM signals. In examples described herein, electrodes 26 can be configured to deliver ablation energy (IRE and/or RF) to tissue in heart 12. In addition to using electrodes 26 to deliver ablation energy, the electrodes 26 can also be used to determine the location of the end effector 100 and/or to measure a physiological property such as local surface electrical potentials at respective locations on tissue in heart 12. The electrodes 26 can be biased such that a greater portion of the electrode 26 faces outwardly from the end effector 100 such that the electrodes 26 deliver a greater amount of electrical energy outwardly away from the end effector 100 (i.e., toward the heart 12 tissue) than inwardly toward the end effector 100.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes 26 on the inner sides of the spines), and then to the blood pool in heart 12.

Catheter 14 may additionally include a position sensor embedded in or near distal tip 28 for tracking position and orientation of distal tip 28. Optionally and preferably, position sensor is a magnetic based position sensor including three magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage direct current (DC) or alternating current (AC) pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

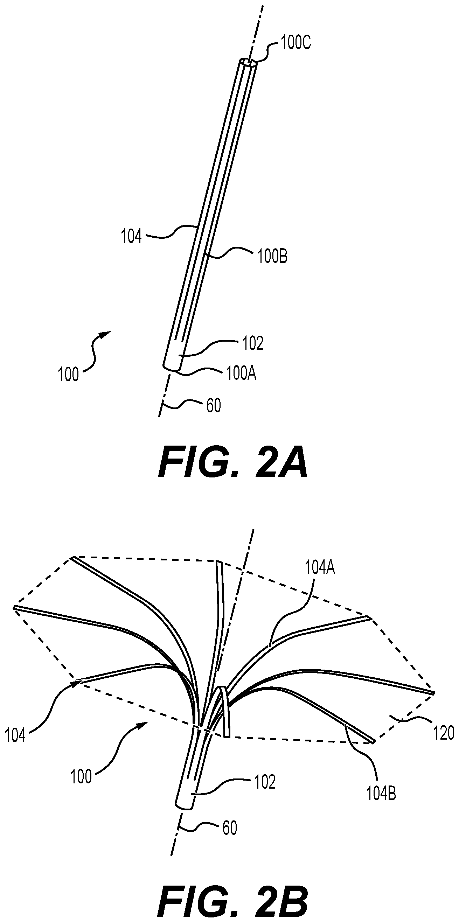

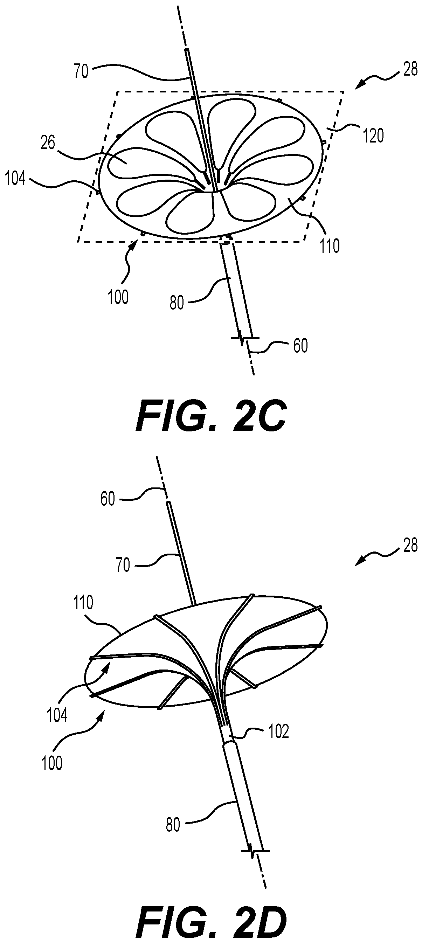

FIG. 2A is a schematic pictorial illustration showing a perspective view of a spine framework 100 of a distal tip 28 of the medical probe 14 in a collapsed configuration. FIG. 2B is a schematic pictorial illustration showing a perspective view of the spine framework 100 in an expanded configuration. FIG. 2C is a schematic pictorial illustration showing a perspective view of a distal side of the distal tip 28, with the spine framework 100, in the expanded configuration. FIG. 2D is a schematic pictorial illustration showing a perspective view of a proximal side of the distal tip of FIG. 2C in the expanded configuration.

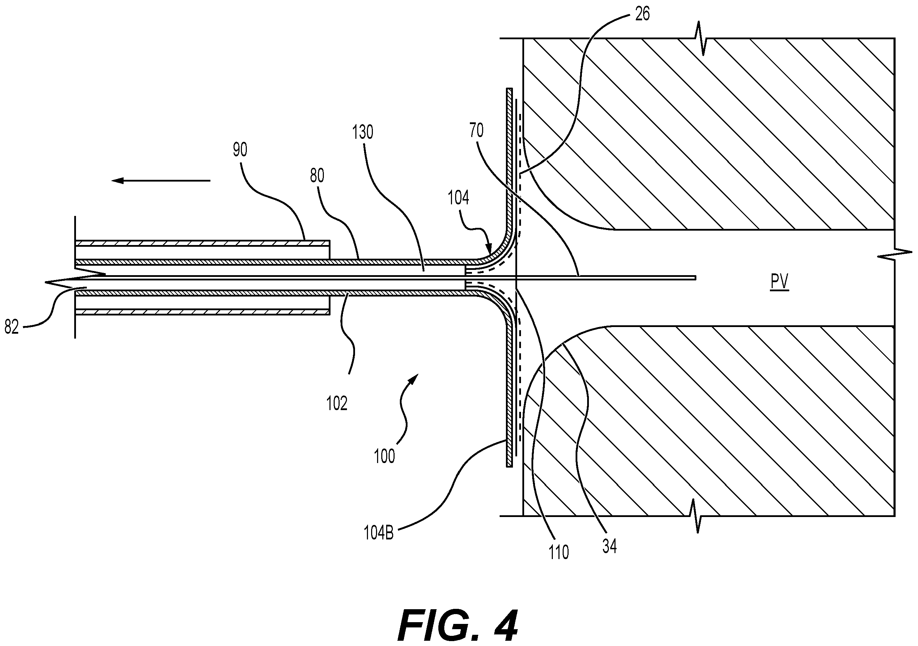

Making reference now to FIGs. 2A and 2B, a distal tip 28 of the catheter/medical probe 14 can be defined by a spine framework 100 that extends along a longitudinal axis 60 and has a proximal end 100A, an intermediate section 100B, and a distal end 100C. The framework 100 includes a neck 102 (that extends from the proximal end 100A) and a plurality of spines 104 (that extend along the intermediate section 100B to the distal end 100C) connected to the neck 102 and extending therefrom. Moreover, the framework 100 defines a lumen 130 (see FIG. 4) that is coaxial with the longitudinal axis 60.

In some examples, the framework 100 is unitary (i.e., a monolithic structure). The framework 100 can be formed from a planar or cylindrical tube stock of material using any suitable method. For example, the framework 100 can be formed by cutting, laser cutting, stamping, combinations thereof, etc. such that it is split to form the spines 104 and define the neck 102.

The framework 100 includes a flexible, resilient material (e.g., a shape-memory alloy such as nickel-titanium, also known as Nitinol) that is shape set to be biased to curve outwardly to an expanded configuration, as shown in FIG. 2B. In the expanded configuration, the framework 100 resembles a lilium flower. Due to the flexible, resilient nature of the material, the spines 104 can also be moved to a collapsed configuration (as shown in FIG. 2A) where the spines 104 extend along the longitudinal axis 60. In the expanded configuration, each spine 104 has a curved section 104A that bends away from the longitudinal axis 60 and a planar section 104B that linearly extends from the curved section 104A along a plane 120 and terminates at the distal end 100C of each spine 104. As seen in FIG. 2B, the plane 120 is approximately orthogonal to the longitudinal axis 60.

Turning now to FIGs. 2C and 2D, to form the distal tip 28 of the probe 14, the spine framework 100 is connected to a flexible elongated shaft 80. A flexible membrane 110 is also connected to the spine framework 100 on a side of the spines 104 that faces distally (relative to a proximal end of the probe 14) in the expanded configuration and inwardly in the collapsed configuration. More particularly, a first portion of the flexible membrane 110 is connected to the planar sections 104B of each spine 104 (so as to form a planar surface that is co-planar with plane 120) and a second portion of the membrane 110 is further connected to the arcuate sections 104A of each spine (with that portion of the membrane 110 resembling a curved funnel in appearance). In some examples, the flexible membrane 110 is made from a biocompatible elastomeric material.

As seen particularly in FIG. 2C, a plurality of electrodes 26 are formed on the distalfacing surface of the flexible membrane 110 that are shaped to maximize contact of the affected area of the heart 12. More specifically, the electrodes 26 are formed at least on the first portion of the flexible membrane 110 such that at least a portion of each electrode 26 is disposed on or are parallel with the plane 120. The electrodes 26 shown in this example may be made, for example, using a conductive epoxy, with a flexible printed circuit board (PCB), or by a vapor deposition layer.

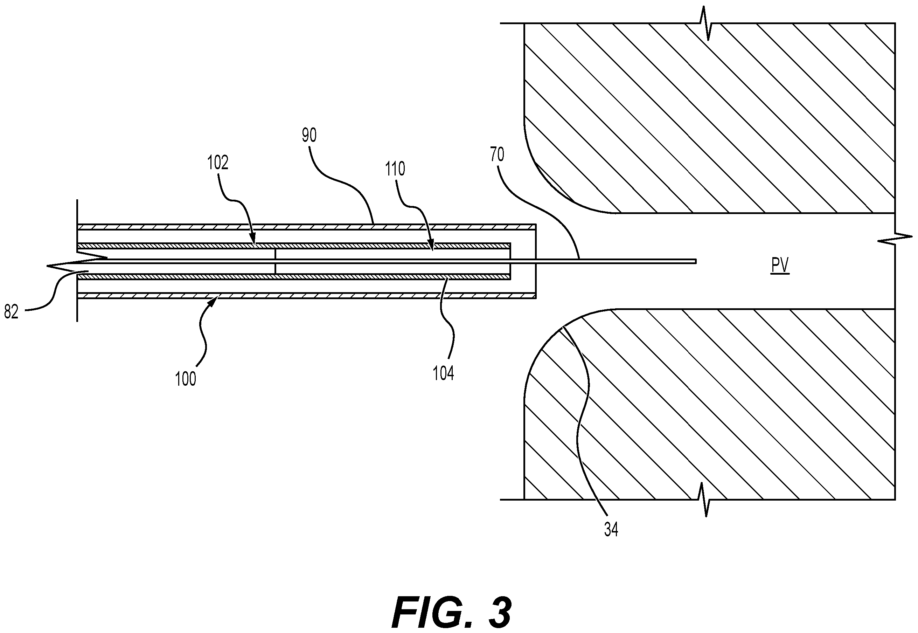

FIG. 3 is a schematic pictorial illustration showing a sectional view of the distal tip 28 of FIG. 2C in the collapsed configuration and proximal an ostium 34 of a pulmonary vein PV. FIG. 4 is a schematic pictorial illustration showing a sectional view of the distal tip 28 of FIG. 2C in the expanded configuration and pressed against the ostium 34 of the pulmonary vein PV.

Making specific reference to FIGs. 3-4, in addition to the medical probe 14, a system in accordance with the presently disclosed technology further includes a guidewire 70 and a guiding sheath 90. The guidewire 70 is routed through the framework lumen 130 of the distal tip 28, through a lumen 82 of the elongated shaft 80, to a proximal end of the medical probe (which includes, e.g., a handle). The guiding sheath 90 is slidable relative to the distal tip 28 of the medical probe 14, and is described in greater detail below.

The following description assumes, by way of example, that a target region (such as an ostium 34 of the pulmonary vein PV) is to be ablated. Of course, it will be appreciated that the presently described technology may be employed to ablate and/or map other regions of the heart 12.

In an initial step, physician 24 inserts the distal tip 28 and guidewire 70 into the subject, with the guiding sheath 90 surrounding the distal tip 28 in a manner that forces the spine framework 100 into the collapsed configuration (from their shape set expanded configuration). The guidewire 70 is then navigated into heart 12. In the present example, within the heart 12, the guidewire 70 is navigated into the pulmonary vein PV.

After the guidewire 70 has been routed to the pulmonary vein PV, the spine framework 100, elongated shaft 80, and guiding sheath 90 are slid along the guidewire 70 until they are positioned near the ostium 34. Once the distal tip 28 of the probe is appropriately positioned, the guiding sheath 90 can be actuated in a proximal direction (leftwards relative to the orientation shown in FIG. 4, and as denoted by the directional arrow). This actuation exposes the distal tip 28, which results in the spines 104 bending outwardly to their shape set form (i.e., the expanded configuration). Once in the expanded configuration, the distally-facing planar face of the flexible membrane 110, which includes the electrodes 26, is pressed against the ostium 34. The planar contact surface of the flexible membrane 110 and the electrodes 26 maximizes the contact area with the ostium 34.

In an ablation step, the physician 24 operates processor 55 and ablation energy generator 50 to supply electrical current to electrodes 26 of the flexible membrane 110. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., patch 38 or the like) coupled to the patient's body.

In some examples, RF sinusoidal-like (alternating) electrical current is supplied to the electrodes 26, such that RF ablation of the tissue is performed. Alternatively, pulsed current (e.g., DC or AC) may be supplied so as to perform irreversible electroporation (IRE) or pulsed field ablation (PFA). When pulsed current is supplied to perform PFA, unipolar ablation energy can be supplied by electrical current running between the electrodes 26 on the flexible membrane 110 and the electrode patch(es) 38 or a backpatch. Moreover, bipolar ablation energy can be supplied by electrical current running between the electrodes 26 on flexible membrane 110 themselves and/or another catheter within another portion of the heart 12.

Depending on placement and arrangement of the electrodes 26 on the flexible membrane 110, a generally full 360-degree band of ablation can be achieved around the ostium 34 to provide pulmonary vein isolation. The electrodes 26 may function therapeutically, for example, for ablating the ostium, with each electrode ablating a predetermined area around the circumference of the ostium 34 to isolate the pulmonary vein without needing to reposition the distal tip 28 one or more times during the treatment.

Optionally, the electrodes 26 may also be used to aid in locating the distal tip 28 and/or sense anatomical signals at the ostium 34. For example, the location of electrodes 26 can be verified by a current tracking module of the PIU 30 using impedances and/or currents between the electrodes 26 and patches 38. Additionally, the location of the electrodes can be verified fluoroscopically. Once the electrodes 26 are positioned properly in contact with the ostium 34, the electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

After the ablating and/or mapping is complete, the physician 24 can slightly retract the distal tip 28 from the ostium and slide the guiding sheath 90 back over the distal tip 28, which causes the spines 104 to bend inwardly from the expanded configuration to the collapsed configuration. Once the spines 104 are collapsed, the guiding sheath 90, medical probe 14, and guidewire 70 can be retracted from the patient's body. Of course, while a guiding sheath 90 has been described as the device that causes the expansion and collapse of the spine framework 100, it will be appreciated that other forms of actuating the spine framework 100 between the expanded and collapsed configuration (and vice versa) may be employed without departing from the spirt and scope of the present disclosure.

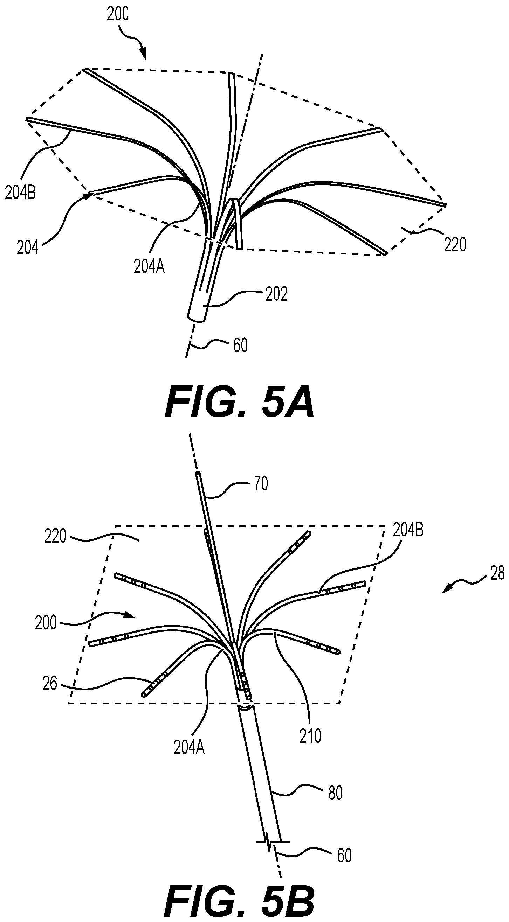

FIG. 5A is a schematic pictorial illustration showing a perspective view of another spine framework 200 of another distal tip 28 of the medical probe 14 in the expanded configuration. FIG. 5B is a schematic pictorial illustration showing a perspective view of a distal side of the distal tip 28, with the spine framework 200 of FIG. 5A, of the medical probe 14 in the expanded configuration.

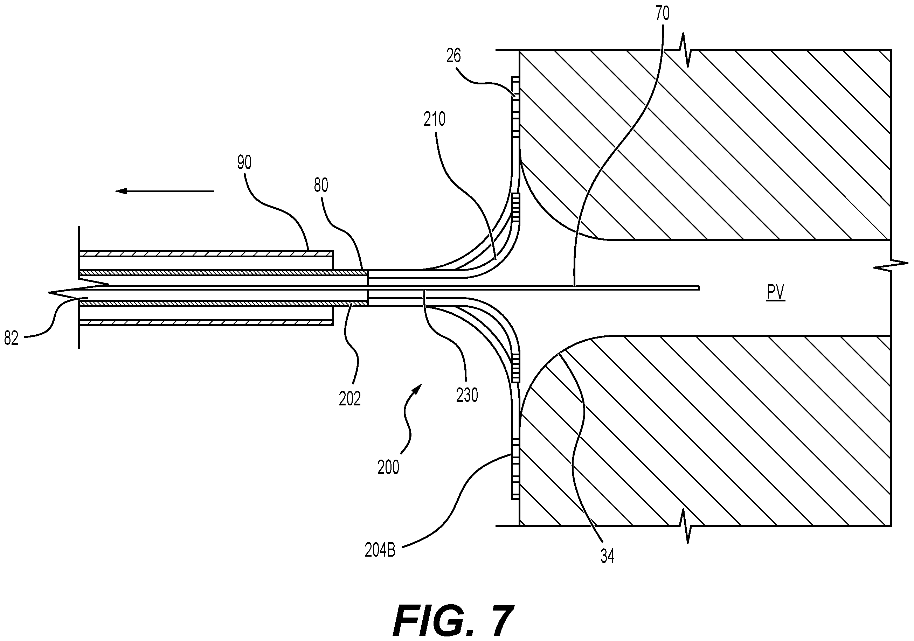

Making reference now to FIG. 5A, another exemplary distal tip 28 of the catheter/medical probe 14 can be defined by a spine framework 200 that extends along a longitudinal axis 60 and has a proximal end, an intermediate section, and a distal end (similar to the previously described framework 100). As seen in FIG. 5A, the framework 200 can be formed similar to or the same as framework 100. The framework 200 includes a neck 202 (that extends from the proximal end) and a plurality of spines 204 (that extend along the intermediate section to the distal end) connected to the neck 102 and extending therefrom. Moreover, the framework 200 defines a lumen 230 (see FIG. 7) that is coaxial with the longitudinal axis 60.

In some examples, the framework 200 is unitary (i.e., a monolithic structure). The framework 200, like the previously described example, can be formed from a planar or cylindrical tube stock of material using any suitable method. For example, the framework 200 can be formed by cutting, laser cutting, stamping, combinations thereof, etc. such that it is split to form the spines 204 and define the neck 202.

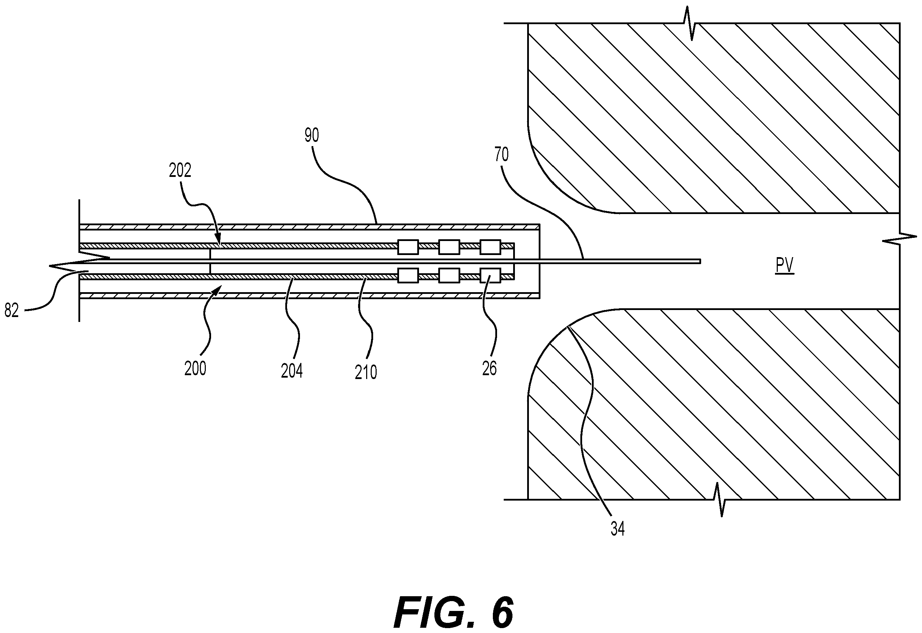

The framework 200 includes a flexible, resilient material (e.g., a shape-memory alloy such as nickel-titanium, also known as Nitinol) that is shape set to be biased to curve outwardly to an expanded configuration, as shown in FIG. 5B. In the expanded configuration, the framework 200 resembles a lilium flower. Due to the flexible, resilient nature of the material, the spines 204 can also be moved to a collapsed configuration (as shown in FIG. 6) where the spines 204 extend along the longitudinal axis 60. In the expanded configuration, each spine 204 has a curved section 204A that bends away from the longitudinal axis 60 and a planar section 204B that linearly extends from the curved section 204A along a plane 220 and terminates at the distal end of each spine 204. As seen in FIG. 5A, the plane 220 is approximately orthogonal to the longitudinal axis 60.

Turning now to FIG. 5B, to form the distal tip 28 of the probe 14, the spine framework 100 is connected to a flexible elongated shaft 80. Moreover, a plurality of electrodes 26 are formed on or around each spine 204 to maximize contact of the affected area of the heart 12 (discussed in greater detail below). More specifically, the electrodes 26 are formed at least on the planar portion 204B of the spines 204 such that at least a portion of each electrode 26 is disposed on or are parallel with the plane 220. The electrodes 26 shown in this example may be made, for example, using a conductive epoxy, with a flexible printed circuit board (PCB), by a vapor deposition layer, or with a ring electrode.

A flexible membrane 210 can also be attached to surround each spine 204. In some examples, the flexible membrane 210 is made from a biocompatible insulative material.

FIG. 6 is a schematic pictorial illustration showing a sectional view of the distal tip 28 of FIG. 5B in the collapsed configuration and proximal an ostium 34 of a pulmonary vein PV. FIG. 7 is a schematic pictorial illustration showing a sectional view of the distal tip 28 of FIG. 5B in the expanded configuration and pressed against the ostium 34 of the pulmonary vein PV.

Making specific reference to FIGs. 6-7, in addition to the medical probe 14, a system in accordance with the presently disclosed technology further includes a guidewire 70 and a guiding sheath 90. The guidewire 70 is routed through the framework lumen 230 of the distal tip 28, through a lumen 82 of the elongated shaft 80, to a proximal end of the medical probe (which includes, e.g., a handle). The guiding sheath 90 is slidable relative to the distal tip 28 of the medical probe 14, and is described in greater detail below.

The following description assumes, by way of example, that a target region (such as an ostium 34 of the pulmonary vein PV) is to be ablated. Of course, it will be appreciated that the presently described technology may be employed to ablate and/or map other regions of the heart 12.

In an initial step, physician 24 inserts the distal tip 28 and guidewire 70 into the subject, with the guiding sheath 90 surrounding the distal tip 28 in a manner that forces the spine framework 200 into the collapsed configuration (from their shape set expanded configuration). The guidewire 70 is then navigated into heart 12. In the present example, within the heart 12, the guidewire 70 is navigated into the pulmonary vein PV.

After the guidewire 70 has been routed to the pulmonary vein PV, the spine framework 100, elongated shaft 80, and guiding sheath 90 are slid along the guidewire 70 until they are positioned near the ostium 34. Once the distal tip 28 of the probe 14 is appropriately positioned, the guiding sheath 90 can be actuated in a proximal direction (leftwards relative to the orientation shown in FIG. 7, and as denoted by the directional arrow). This actuation exposes the distal tip 28, which results in the spines 204 bending outwardly to their shape set form (i.e., the expanded configuration). Once in the expanded configuration, the distally-facing planar sections 204B of the spines 204, which includes the electrodes 26, are pressed against the ostium 34. The planar contact surface 220, which the electrodes 26 are disposed thereon, maximizes the contact area with the ostium 34.

In an ablation step, the physician 24 operates processor 55 and ablation energy generator 50 to supply electrical current to electrodes 26 on the spines 204. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., patch 38 or the like) coupled to the patient's body.

In some examples, RF sinusoidal-like (alternating) electrical current is supplied to the electrodes 26, such that RF ablation of the tissue is performed. Alternatively, pulsed current (e.g. DC or AC) may be supplied so as to perform irreversible electroporation (IRE) or pulsed field ablation (PFA). When pulsed current is supplied to perform PFA, unipolar ablation energy can be supplied by electrical current running between the electrodes 26 on the spines 204 and the electrode patch(es) 38 or a backpatch. Moreover, bipolar ablation energy can be supplied by electrical current running between the electrodes 26 themselves and/or another catheter within another portion of the heart 12.

Depending on placement and arrangement of the electrodes 26 on the spines 204, a generally full 360-degree band of ablation can be achieved around the ostium 34 to provide pulmonary vein isolation. The electrodes 26 may function therapeutically, for example, for ablating the ostium, with each electrode ablating a predetermined area around the circumference of the ostium 34 to isolate the pulmonary vein without needing to reposition the distal tip 28 one or more times during the treatment.

Optionally, the electrodes 26 may also be used to aid in locating the distal tip 28 and/or sense anatomical signals at the ostium 34. For example, the location of electrodes 26 can be verified by a current tracking module of the PIU 30 using impedances and/or currents between the electrodes 26 and patches 38. Additionally, the location of the electrodes can be verified fluoroscopically. Once the electrodes 26 are positioned properly in contact with the ostium 34, the electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

After the ablating and/or mapping is complete, the physician 24 can slightly retract the distal tip 28 from the ostium and slide the guiding sheath 90 back over the distal tip 28, which causes the spines 204 to bend inwardly from the expanded configuration to the collapsed configuration. Once the spines 204 are collapsed, the guiding sheath 90, medical probe 14, and guidewire 70 can be retracted from the patient's body. Of course, while a guiding sheath 90 has been described as the device that causes the expansion and collapse of the spine framework 200, it will be appreciated that other forms of actuating the spine framework 200 between the expanded and collapsed configuration (and vice versa) may be employed without departing from the spirt and scope of the present disclosure.

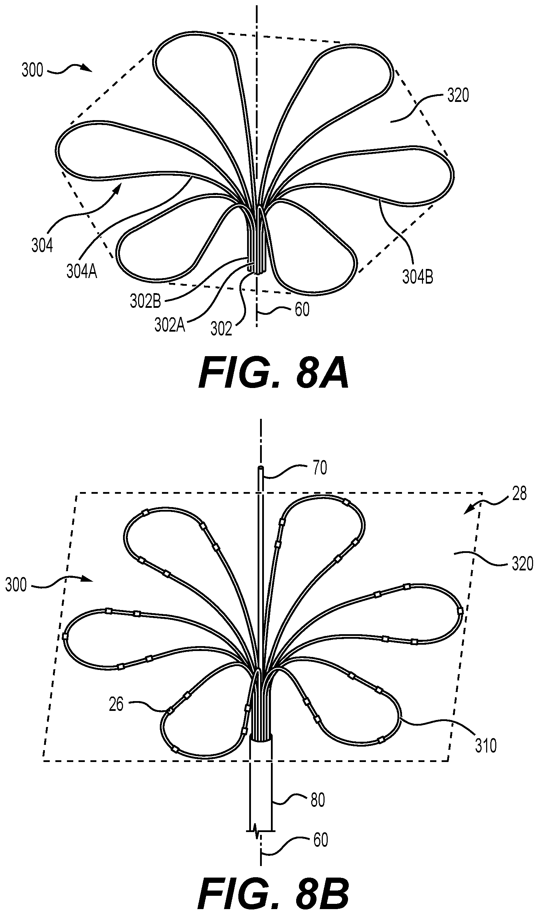

FIG. 8A is a schematic pictorial illustration showing a perspective view of another spine framework 300 of yet another exemplary distal tip 28 of the medical probe 14 in the expanded configuration. FIG. 8B is a schematic pictorial illustration showing a perspective view of a distal side of the distal tip 28, with the spine framework 300 of FIG. 8A, of the medical probe 14 in the expanded configuration.

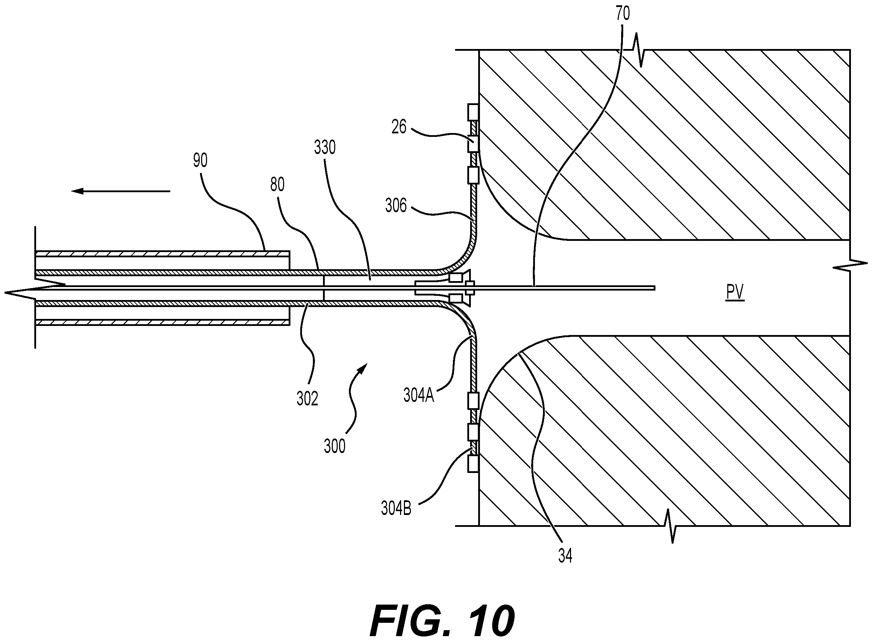

Making reference now to FIG. 8A, another exemplary distal tip 28 of the catheter/medical probe 14 can be defined by a spine framework 300 that extends along a longitudinal axis 60 and has a proximal end, an intermediate section, and a distal end (similar to the previously described frameworks 100, 200). As seen in FIG. 8A, the framework 300 can be formed to take a similar shape as that of frameworks 100, 200 in the expanded configuration. The framework 300 includes a neck 302 (that extends from the proximal end) and a plurality of spines 304 (that extend along the intermediate section to the distal end) connected to the neck 302 and extending therefrom. Moreover, the framework 300 defines a lumen 330 (see FIG. 10) that is coaxial with the longitudinal axis 60.

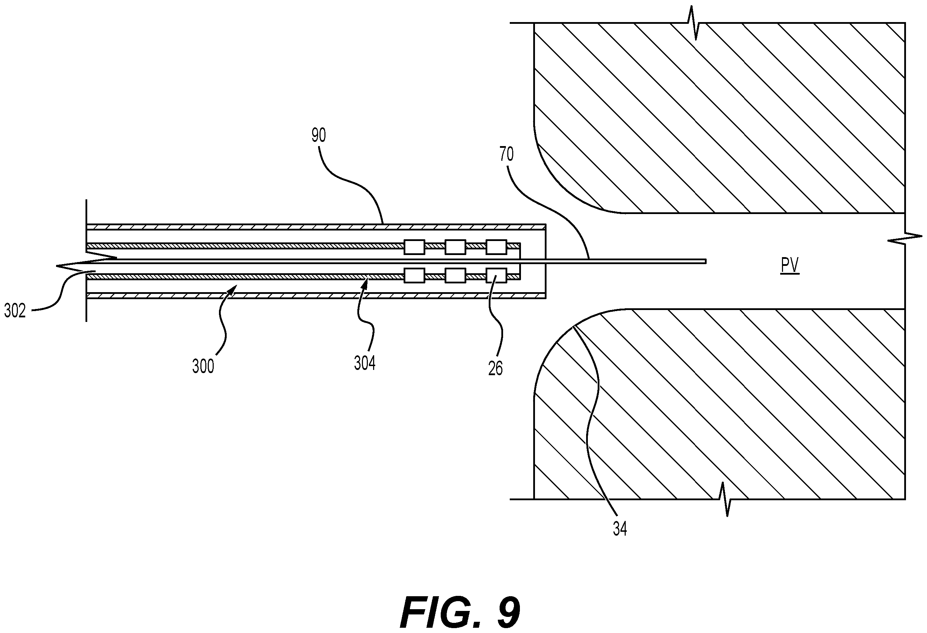

In some examples, the framework 300 is formed from a series of flexible, resilient rods or tubes (e.g., formed from a shape-memory alloy such as Nitinol) that each include a first end 302A and a second end 302B that define portions of the neck 302 (all of the first ends 302A and second ends 302B, when assembled/connected together, conjunctively form the neck 302 that defines the lumen 330). Each rod is bent to include a loop portion 304 that form the spines 304 of the framework 300 and shape set to be biased to curve outwardly to an expanded configuration, as shown in FIG. 8A. In the expanded configuration, each bent rod resembles a flower pedal. Due to the flexible, resilient nature of the material, the spines 304 can also be moved to a collapsed configuration (as shown in FIG. 9) where the spines 304 extend along the longitudinal axis 60.

In the expanded configuration, each loop portion 304 has two curved sections 304A that bend away from the longitudinal axis 60 and a planar section 304B that linearly extends from the curved sections 304A along a plane 320 and loops around at the distal end of each spine 304. In other words, the planar section 304B loops from the first end 302A towards the second end 302B and connects the curved sections 304A, forming a partial loop shape. As seen in FIG. 8A, the plane 320 is approximately orthogonal to the longitudinal axis 60.

Turning now to FIG. 8B, to form the distal tip 28 of the probe 14, the spine framework 300 is connected to a flexible elongated shaft 80. Moreover, a plurality of electrodes 26 are formed on or around the planar section 304B of each spine 304 to maximize contact of the affected area of the heart 12 (discussed in greater detail below). More specifically, the electrodes 26 are formed on the spines 304 such that at least a portion of each electrode 26 is disposed on or are parallel with the plane 320. The electrodes 26 shown in this example may be made, for example, using a conductive epoxy, with a flexible printed circuit board (PCB), by a vapor deposition layer, or with a ring electrode.

A flexible membrane 310 can also be attached to surround each spine 304 and the neck 302. In some examples, the flexible membrane 310 is made from a biocompatible insulative material.

FIG. 9 is a schematic pictorial illustration showing a sectional view of the distal tip 28 of FIG. 8B in the collapsed configuration and proximal an ostium 34 of a pulmonary vein PV. FIG. 10 is a schematic pictorial illustration showing a sectional view of the distal tip 28 of FIG. 8B in the expanded configuration and pressed against the ostium 34 of the pulmonary vein PV.

Making specific reference to FIGs. 9-10, in addition to the medical probe 14, a system in accordance with the presently disclosed technology further includes a guidewire 70 and a guiding sheath 90. The guidewire 70 is routed through the framework lumen 330 of the distal tip 28, through a lumen 82 of the elongated shaft 80, to a proximal end of the medical probe (which includes, e.g., a handle). The guiding sheath 90 is slidable relative to the distal tip 28 of the medical probe 14, and is described in greater detail below.

The following description assumes, by way of example, that a target region (such as an ostium 34 of the pulmonary vein PV) is to be ablated. Of course, it will be appreciated that the presently described technology may be employed to ablate and/or map other regions of the heart 12.

In an initial step, physician 24 inserts the distal tip 28 and guidewire 70 into the subject, with the guiding sheath 90 surrounding the distal tip 28 in a manner that forces the spine framework 300 into the collapsed configuration (from their shape set expanded configuration). The guidewire 70 is then navigated into heart 12. In the present example, within the heart 12, the guidewire 70 is navigated into the pulmonary vein PV.

After the guidewire 70 has been routed to the pulmonary vein PV, the spine framework 300, elongated shaft 80, and guiding sheath 90 are slid along the guidewire 70 until they are positioned near the ostium 34. Once the distal tip 28 of the probe 14 is appropriately positioned, the guiding sheath 90 can be actuated in a proximal direction (leftwards relative to the orientation shown in FIG. 10, and as denoted by the directional arrow). This actuation exposes the distal tip 28, which results in the spines 304 bending outwardly to their shape set form (i.e., the expanded configuration). Once in the expanded configuration, the distally-facing planar sections 304B of the spines 304, which includes the electrodes 26, are pressed against the ostium 34, as shown in FIG. 10. The planar contact surface 320, which the electrodes 26 are disposed thereon, maximizes the contact area with the ostium 34.

In an ablation step, the physician 24 operates processor 55 and ablation energy generator 50 to supply electrical current to electrodes 26 on the spines 304. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., patch 38 or the like) coupled to the patient's body.

In some examples, RF sinusoidal-like (alternating) electrical current is supplied to the electrodes 26, such that RF ablation of the tissue is performed. Alternatively, pulsed current (e.g., DC or AC) may be supplied so as to perform irreversible electroporation (IRE) or pulsed field ablation (PFA). When pulsed current is supplied to perform PFA, unipolar ablation energy can be supplied by electrical current running between the electrodes 26 on the spines 204 and the electrode patch(es) 38 or a backpatch. Moreover, bipolar ablation energy can be supplied by electrical current running between the electrodes 26 themselves and/or another catheter within another portion of the heart 12.

Depending on placement and arrangement of the electrodes 26 on the spines 304, a generally full 360-degree band of ablation can be achieved around the ostium 34 to provide pulmonary vein isolation. The electrodes 26 may function therapeutically, for example, for ablating the ostium, with each electrode ablating a predetermined area around the circumference of the ostium 34 to isolate the pulmonary vein without needing to reposition the distal tip 28 one or more times during the treatment.

Optionally, the electrodes 26 may also be used to aid in locating the distal tip 28 and/or sense anatomical signals at the ostium 34. For example, the location of electrodes 26 can be verified by a current tracking module of the PIU 30 using impedances and/or currents between the electrodes 26 and patches 38. Additionally, the location of the electrodes can be verified fluoroscopically. Once the electrodes 26 are positioned properly in contact with the ostium 34, the electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

After the ablating and/or mapping is complete, the physician 24 can slightly retract the distal tip 28 from the ostium 34 and slide the guiding sheath 90 back over the distal tip 28, which causes the spines 304 to bend inwardly from the expanded configuration to the collapsed configuration. Once the spines 304 are collapsed, the guiding sheath 90, medical probe 14, and guidewire 70 can be retracted from the patient's body. Of course, while a guiding sheath 90 has been described as the device that causes the expansion and collapse of the spine framework 300, it will be appreciated that other forms of actuating the spine framework 300 between the expanded and collapsed configuration (and vice versa) may be employed without departing from the spirt and scope of the present disclosure.

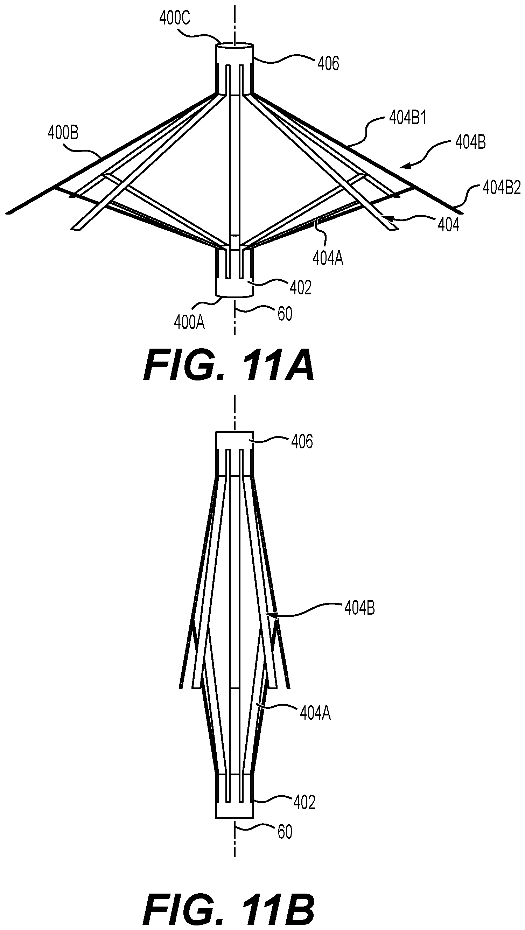

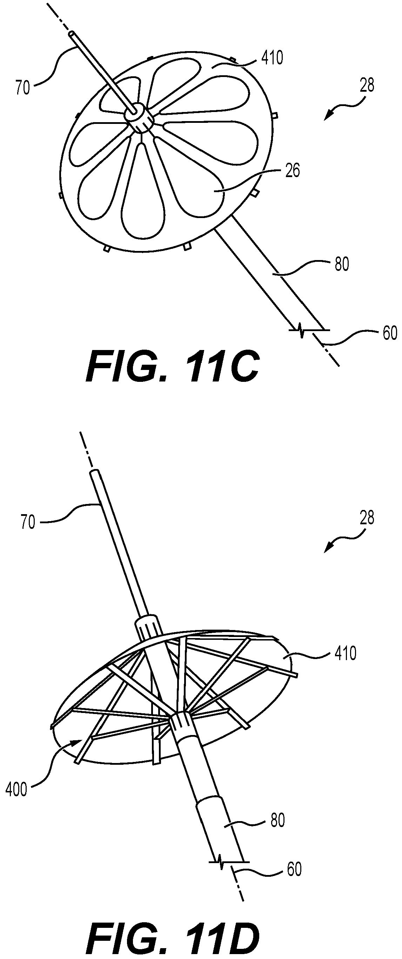

FIG. 11A is a schematic pictorial illustration showing a perspective view of a spine framework 400 of yet another distal tip 28 of the medical probe 14 in an expanded configuration. FIG. 2B is a schematic pictorial illustration showing a perspective view of the spine framework 400 of FIG. 11A in a collapsed configuration. FIG. 2C is a schematic pictorial illustration showing a perspective view of a distal side of the distal tip 28, with the spine framework 400, in the expanded configuration. FIG. 2D is a schematic pictorial illustration showing a perspective view of a proximal side of the distal tip 28 of FIG. 2C, in the expanded configuration.

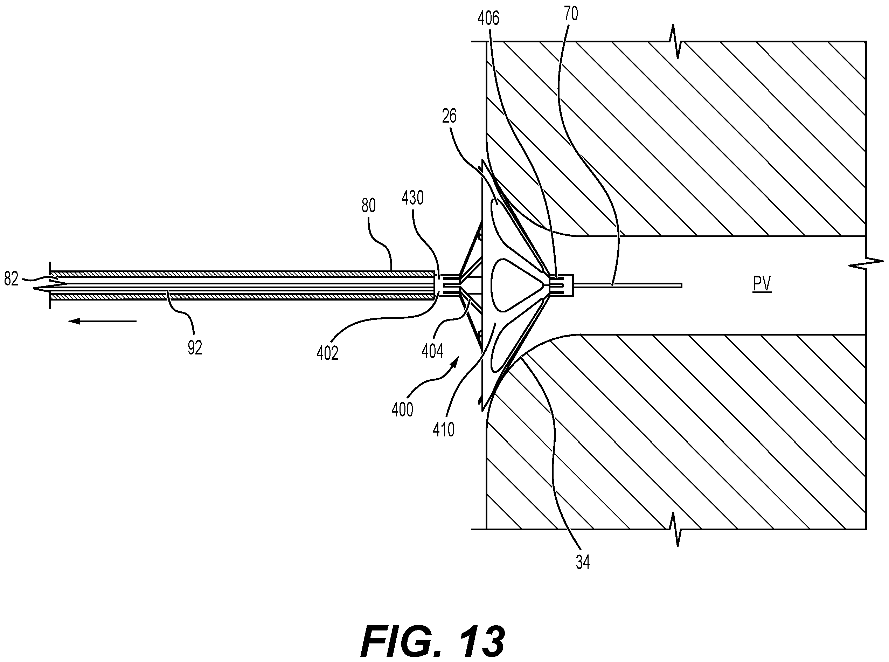

Making reference now to FIG. 11A, another exemplary distal tip 28 of the catheter/medical probe 14 can be defined by a spine framework 400 that extends along a longitudinal axis 60 and has a proximal end 400A, an intermediate section 400B, and a distal end 400C (similar to the previously described frameworks 100, 200, 300). As seen in FIG. 11A, the framework 400 can be formed to take a similar shape as that of a basket catheter. The framework 400 includes a neck 402 (that extends from the proximal end 400A) and a plurality of spines 404 (that extend along the intermediate section 400B) connected to the neck 402 and extending therefrom. The spines 404, at a side opposite the one that connects with the neck 402, connect with a crown 406 that defines the distal end 400C. Moreover, the framework 400 defines a lumen 430 (see FIG. 13) that is coaxial with the longitudinal axis 60.

In some examples, the framework 400 is unitary (i.e., a monolithic structure) and formed form a flexible, resilient material (e.g., Nitinol). The framework 400 can be formed from a planar or cylindrical tube stock of material using any suitable method. For example, the framework 400 can be formed by cutting, laser cutting, stamping, combinations thereof, etc. such that it is split to form the spines 404 and define the neck 402 and crown 406. Alternatively, or additionally, the framework 400 can be formed such that a portion 404B2 of the spines 404 extends from a joint section between two pieces 404A, 404B of the spine 404. Due to the resilient nature of the material, the framework 400 can be shape set to be biased to either an expanded configuration, as shown in FIG. 11A or a collapsed configuration, as shown in FIG. 11B.

Further to the above, each spine 404 includes a straight section 404A that is connected to the neck 402 and a second straight section 404B that is connected to the first straight section 404A as a joint that is intermediate opposing ends of the second straight section. In the collapsed configuration, each connected first straight section 404A and second straight section 404B are oriented at a first angle relative to one another. In the expanded configuration, each connected first straight section 404A and second straight section 404B is oriented at a second angle relative to one another, with the second angle being smaller than the first angle (as seen comparing FIG. 11A with FIG. 11B, for example). In the collapsed configuration, each spine 404 extends along the longitudinal axis 60 (but is not necessarily completely parallel thereto). The width of each spine 404 can be adjusted in order to change the stiffness of the framework 400.

Turning now to FIG. 11B, to form the distal tip 28 of the probe 14, the spine framework 300 is connected to a flexible elongated shaft 80 that includes a shaft lumen 82. A flexible membrane 410 is also connected to the spine framework 400 on a portion 404B1 of the second straight section 404B of the spines 104 that faces distally (relative to a proximal end of the probe 14) in the expanded configuration and outwardly in the collapsed configuration. When connected to the spines 404, the membrane 410 and spines 404 generally resemble the form of an umbrella and is approximately cone-shaped. In some examples, the flexible membrane 410 is made from a biocompatible elastomeric material.

Moreover, a plurality of electrodes 26 are formed on a distally facing section of the membrane 420 to, in conjunction with the conical shape of the membrane in the expanded configuration, maximize contact of the affected area of the heart 12 (discussed in greater detail below). The electrodes 26 shown in this example may be made, for example, using a conductive epoxy, with a flexible printed circuit board (PCB), or by a vapor deposition layer.

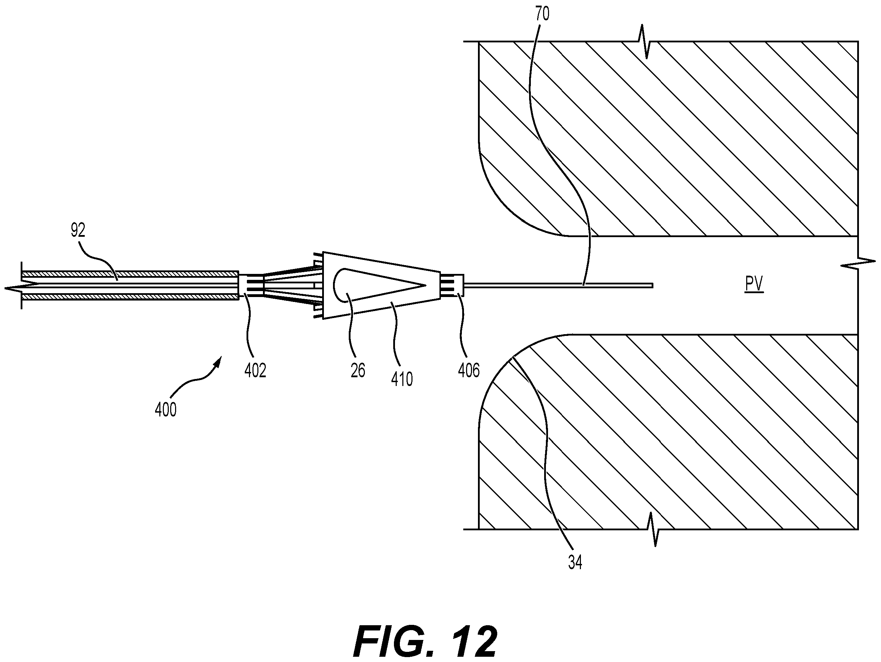

FIG. 12 is a schematic pictorial illustration showing a sectional view of the distal tip 28 of FIG. 11C in the collapsed configuration and proximal an ostium 34 of a pulmonary vein PV. FIG. 13 is a schematic pictorial illustration showing a sectional view of the distal tip 28 of FIG. 11C in the expanded configuration and pressed against the ostium 34 of the pulmonary vein PV.

Making specific reference to FIGs. 12-13, in addition to the medical probe 14, a system in accordance with the presently disclosed technology further includes a guidewire 70 and an actuator 92 (embodied as a push/pull rod in the present example, but may take other forms, such as a pull wire). The guidewire 70 is routed through the framework lumen 430 of the distal tip 28, through the lumen 82 of the elongated shaft 80, to a proximal end of the medical probe (which includes, e.g., a handle). The push rod is connected to either the crown 406 or the neck 402 of the spine framework 400, and is described in greater detail below.

The following description assumes, by way of example, that a target region (such as an ostium 34 of the pulmonary vein PV) is to be ablated. Of course, it will be appreciated that the presently described technology may be employed to ablate and/or map other regions of the heart 12.

In an initial step, physician 24 inserts the distal tip 28 and guidewire 70 into the subject, with the push rod 92 being positioned to urge the spine framework 400 into the collapsed configuration. For example, when connected to the crown 406, a distally directed force (i.e., rightwards relative to FIG. 12) on the push rod 92 maintains the framework 400 in a collapsed configuration, whereas when connected to the neck 402, the neck 402 can be made slidable such that a proximally directed force (i.e., leftwards relative to FIG. 13, as denoted by the directional arrow) on the push rod 92 maintains the framework 400 in a collapsed configuration. The guidewire 70 is then navigated into heart 12. In the present example, within the heart 12, the guidewire 70 is navigated into the pulmonary vein PV.

After the guidewire 70 has been routed to the pulmonary vein PV, the spine framework 400, elongated shaft 80, and push rod 92 are slid along the guidewire 70 until they are positioned near the ostium 34. Once the distal tip 28 of the probe 14 is appropriately positioned, the push rod 92 (when connected to the crown 406) can be actuated in the proximal direction (leftwards relative to the orientation shown in FIG. 13, and, as mentioned above, as denoted by the directional arrow). This actuation exposes the distal tip 28, which results in the spines 304 bending outwardly to the expanded configuration. Once in the expanded configuration, the conical form of the membrane 410, which includes the electrodes 26, is optimized to maximize the contact area when pressed against the ostium 34, as shown in FIG. 13.

In an ablation step, the physician 24 operates processor 55 and ablation energy generator 50 to supply electrical current to electrodes 26 on the membrane 410. If there are two or more electrodes 26, then the supplied current can be bipolar, i.e., the current can be passed between the electrodes 26 so as to convey ablation energy to the tissue. Alternatively, the supplied ablation energy can be unipolar, i.e., an electrical current may be applied between one of electrodes 26 and a return electrode connected to generator 50. The return electrode can be disposed outside the body of patient 23. For example, the return electrode may comprise a patch (e.g., patch 38 or the like) coupled to the patient's body.

In some examples, RF sinusoidal-like (alternating) electrical current is supplied to the electrodes 26, such that RF ablation of the tissue is performed. Alternatively, pulsed current (e.g., DC or AC) may be supplied so as to perform irreversible electroporation (IRE) or pulsed field ablation (PFA). When pulsed current is supplied to perform PFA, unipolar ablation energy can be supplied by electrical current running between the electrodes 26 on the spines 204 and the electrode patch(es) 38 or a backpatch. Moreover, bipolar ablation energy can be supplied by electrical current running between the electrodes 26 themselves and/or another catheter within another portion of the heart 12.

Depending on placement and arrangement of the electrodes 26 on the membrane 410, a generally full 360-degree band of ablation can be achieved around the ostium 34 to provide pulmonary vein isolation. The electrodes 26 may function therapeutically, for example, for ablating the ostium, with each electrode ablating a predetermined area around the circumference of the ostium 34 to isolate the pulmonary vein without needing to reposition the distal tip 28 one or more times during the treatment.

Optionally, the electrodes 26 may also be used to aid in locating the distal tip 28 and/or sense anatomical signals at the ostium 34. For example, the location of electrodes 26 can be verified by a current tracking module of the PIU 30 using impedances and/or currents between the electrodes 26 and patches 38. Additionally, the location of the electrodes can be verified fluoroscopically. Once the electrodes 26 are positioned properly in contact with the ostium 34, the electrodes 26 generate potential gradient signals in response to the sensed electrical potentials, also referred to herein as electrical signals. In some examples, the sensed electrical signals are indicative of at least one characteristic of the anatomical signals, such as a direction and propagation speed of wavefronts caused by anatomical signals, such as electrocardiogram (ECG) signals in the heart 12.

After the ablating and/or mapping is complete, the physician 24 can slightly retract the distal tip 28 from the ostium 34 and slide the push rod 92 distally to cause the spines 404 to bend from the expanded configuration to the collapsed configuration. Once the spines 404 are collapsed, the guiding sheath 90, medical probe 14, and guidewire 70 can be retracted from the patient's body. Of course, while a push rod 92 has been described as the device that causes the expansion and collapse of the spine framework 400, it will be appreciated that other forms of actuating the spine framework 400 between the expanded and collapsed configuration (and vice versa) may be employed without departing from the spirt and scope of the present disclosure.

The disclosed technology described herein can be further understood according to the following clauses:
Clause 1. A distal tip of a medical probe, the distal tip comprising a spine framework having a proximal end, an intermediate section, and a distal end and extending along a longitudinal axis, the spine framework comprising: a neck extending from the proximal end; and a plurality of spines connected to the neck and extending along the intermediate section to the distal end, the spines being movable between: a collapsed configuration, where the spines extend along the longitudinal axis; and an expanded configuration, where each spine comprises a curved section that bends away from the longitudinal axis and a planar section extending from the curved section to the distal end, each planar section extending along a plane that is approximately orthogonal to the longitudinal axis.
Clause 2. The distal tip of clause 1, each spine extending approximately parallel to the longitudinal axis in the collapsed configuration.
Clause 3. The distal tip of any one of clauses 1-2, the spine framework defining a lumen therethrough.
Clause 4. The distal tip of any one of clauses 1-3, the spine framework comprising a shape-memory alloy material.
Clause 5. The distal tip of any one of clauses 1-4, further comprising a plurality of electrodes connected to the spine framework.
Clause 6. The distal tip of clause 5, each electrode comprising at least one of a conductive epoxy, a flexible printed circuit board, a vapor deposition layer, or a ring electrode.
Clause 7. The distal tip of any one of clauses 1-4, each planar section terminating at the distal end.
Clause 8. The distal tip of clause 7, further comprising a flexible membrane connected to the planar sections of the spines, the flexible membrane comprising a first portion that extends along the plane in the expanded configuration.
Clause 9. The distal tip of clause 8, the flexible membrane further being connected to the arcuate sections of the spines.
Clause 10. The distal tip of any one of clauses 8-9, further comprising one or more electrodes connected to the first portion of the flexible membrane.
Clause 11. The distal tip of clause 7, further comprising a plurality of flexible membranes, each flexible membrane surrounding a respective spine.
Clause 12. The distal tip of clause 11, further comprising one or more ring electrodes connected to each spine.
Clause 13. The distal tip of any one of clauses 1-4, the spine framework comprising a plurality of rods, each rod comprising: a first end; a second end; and a loop portion connecting the first end and the second end, the first ends and the second ends of the rods conjunctively forming the neck, and the loop portions forming the spines.
Clause 14. The distal tip of clause 13, further comprising a plurality of flexible membranes, each flexible membrane surrounding a respective spine.
Clause 15. The distal tip of clause 14, further comprising one or more ring electrodes connected to each spine.
Clause 16. A distal tip of a medical probe, the distal tip comprising a spine framework having a proximal end, an intermediate section, and a distal end and extending along a longitudinal axis, the spine framework comprising: a neck extending from the proximal end; a plurality of spines extending along the intermediate section, each spine comprising: a first straight section connected to the neck; and a second straight section comprising a first end and a second end, the first straight section being connected to the second straight section at a joint intermediate the first end and the second end; a crown connected to the second straight section and extending to the distal end, the spine framework being moveable between: a collapsed configuration, where each connected first straight section and second straight section are oriented at a first angle relative to one another; and an expanded configuration, where each connected first straight section and second straight section are oriented at a second angle relative to one another, the second angle being smaller than the first angle.
Clause 17. The distal tip of clause 16, further comprising a flexible membrane connected to the second straight sections.
Clause 18. The distal tip of clause 17, further comprising one or more electrodes connected to the flexible membrane.
Clause 19. The distal tip of clause 18, each electrode comprising at least one of a conductive epoxy, a flexible printed circuit board, or a vapor deposition layer.
Clause 20. The distal tip of any one of clauses 17-19, the flexible membrane comprising a conical shape in the expanded configuration of the spine framework.
Clause 21. The distal tip of any one of clauses 16-20, each spine extending along the longitudinal axis in the collapsed configuration.
Clause 22. The distal tip of any one of clauses 16-21, the spine framework defining a lumen therethrough.
Clause 23. The distal tip of any one of clauses 16-22, the spine framework comprising a shape-memory alloy material.
Clause 24. A system comprising: a medical probe comprising: an elongated shaft extending along a longitudinal axis; and a distal tip of a medical probe, the distal tip comprising a spine framework having a proximal end, an intermediate section, and a distal end and extending along a longitudinal axis, the spine framework comprising: a neck extending from the proximal end; and a plurality of spines connected to the neck and extending along the intermediate section to the distal end, the spines being movable between: a collapsed configuration, where the spines extend along the longitudinal axis; and an expanded configuration, where each spine comprises a curved section that bends away from the longitudinal axis and a planar section extending from the curved section to the distal end, each planar section extending along a plane that is approximately orthogonal to the longitudinal axis; a guidewire extending through the elongated shaft and the distal tip; and a guiding sheath that is slidable relative to the distal tip to cause the spines to move between the collapsed configuration and the expanded configuration.
Clause 25. The system of clause 24, the elongated shaft defining a lumen and the spine framework defining a lumen, the guidewire extending through the lumens.
Clause 26. The system of any one of clauses 24-25, further comprising a plurality of electrodes connected to the spine framework.
Clause 27. The system of any one of clauses 24-27, the spines being biased to the expanded configuration.
Clause 28. A system comprising: a medical probe comprising: an elongated shaft extending along a longitudinal axis; and a distal tip comprising a spine framework having a proximal end, an intermediate section, and a distal end and extending along a longitudinal axis, the spine framework comprising: a neck extending from the proximal end; a plurality of spines extending along the intermediate section, each spine comprising: a first straight section connected to the neck; a second straight section comprising a first end and a second end, the first straight section being connected to the second straight section at a joint intermediate the first end and the second end; and a crown connected to the second straight section and extending to the distal end, the spine framework being moveable between: a collapsed configuration, where each connected first straight section and second straight section are oriented at a first angle relative to one another; and an expanded configuration, where each connected first straight section and second straight section are oriented at a second angle relative to one another, the second angle being smaller than the first angle; a guidewire extending through the elongated shaft and the distal tip; and a rod or wire that is connected to one of the neck or the crown and operable to move the spine framework between the collapsed configuration and the expanded configuration.
Clause 29. The system of clause 28, the elongated shaft defining a lumen and the spine framework defining a lumen, the guidewire extending through the lumens.
Clause 30. The system of any one of clauses 28-29, further comprising a plurality of electrodes connected to the spine framework.
Clause 31. The system of any one of clauses 28-30, the spines being biased to the expanded configuration or the collapsed configuration.
Clause 32. A method of making a distal dip of a medical probe, the method comprising: cutting a tube, that extends along a longitudinal axis, to define a neck and a plurality of spines; and shape setting the spines such that each spine comprises a curved section that bends away the longitudinal axis and a planar section extending from the curved section, each planar section extending along a plane that is approximately orthogonal to the longitudinal axis.
Clause 33. A method of making a distal dip of a medical probe, the method comprising: shape setting a plurality of rods such that each rod comprises: a first end extending along a longitudinal axis; a first curved section extending from the first end and bending away from the longitudinal axis; a planar section extending from the curved section that forms a partial loop shape, the planar section extending along a plane that is approximately orthogonal to the longitudinal axis; a second curved section extending from the planar section and bending towards the longitudinal axis; and a second end extending from the second curved section and extending along the longitudinal axis; and connecting the rods together such that the first ends and the second ends define a lumen.
Clause 34. A method of using a medical probe, the medical probe comprising a distal tip that comprises a spine framework having a proximal end, an intermediate section, and a distal end and extending along a longitudinal axis, the spine framework comprising a neck extending from the proximal end and a plurality of spines connected to the neck and extending along the intermediate section to the distal end, a plurality of electrodes being connected to the spine framework, the method comprising: moving the spines from a collapsed configuration, where the spines extend along the longitudinal axis, to an expanded configuration, where each spine comprises a curved section that bends away the longitudinal axis and a planar section extending from the curved section to the distal end, each planar section extending along a plane that is approximately orthogonal to the longitudinal axis; and positioning the planar sections against a pulmonary vein opening such that the electrodes are placed in contact with the pulmonary vein opening.
Clause 35. A method of using a medical probe, the medical probe comprising a distal tip that comprises a spine framework having a proximal end, an intermediate section, and a distal end and extending along a longitudinal axis, the spine framework comprising a neck extending from the proximal end, a plurality of spines extending along the intermediate section, and a crown connected to the second straight section and extending to the distal end, with each spine comprising a first straight section connected to the neck and a second straight section comprising a first end and a second end, the first straight section being connected to the second straight section at a joint intermediate the first end and the second end, the method comprising: moving spine framework from a collapsed configuration, where each connected first straight section and second straight section are oriented at a first angle relative to one another, to an expanded configuration, where each connected first straight section and second straight section are oriented at a second angle relative to one another, the second angle being smaller than the first angle; and positioning the second straight sections against a pulmonary vein opening such that the electrodes are placed in contact with the pulmonary vein opening.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A distal tip of a medical probe, the distal tip comprising a spine framework having a proximal end, an intermediate section, and a distal end and extending along a longitudinal axis, the spine framework comprising:
a neck extending from the proximal end; and
a plurality of spines connected to the neck and extending along the intermediate section to the distal end, the spines being movable between:
a collapsed configuration, where the spines extend along the longitudinal axis; and
an expanded configuration, where each spine comprises a curved section that bends away from the longitudinal axis and a planar section extending from the curved section to the distal end, each planar section extending along a plane that is approximately orthogonal to the longitudinal axis.

2. The distal tip of claim 1, each spine extending approximately parallel to the longitudinal axis in the collapsed configuration.

3. The distal tip of claim 1 or claim 2, the spine framework defining a lumen therethrough.

4. The distal tip of any preceding claim, further comprising a plurality of electrodes connected to the spine framework.

5. The distal tip of any one of claims 1 to 3, each planar section terminating at the distal end.

6. The distal tip of claim 5, further comprising a flexible membrane connected to the planar sections of the spines, the flexible membrane comprising a first portion that extends along the plane in the expanded configuration.

7. The distal tip of claim 6, the flexible membrane further being connected to the arcuate sections of the spines and/or further comprising one or more electrodes connected to the first portion of the flexible membrane.

8. The distal tip of claim 5, further comprising a plurality of flexible membranes, each flexible membrane surrounding a respective spine, optionally further comprising one or more ring electrodes connected to each spine.

9. The distal tip of any of claims 1 to 3, the spine framework comprising a plurality of rods, each rod comprising:
a first end;
a second end; and
a loop portion connecting the first end and the second end,
the first ends and the second ends of the rods conjunctively forming the neck, and
the loop portions forming the spines.

10. The distal tip of claim 9, further comprising a plurality of flexible membranes, each flexible membrane surrounding a respective spine, optionally further comprising one or more ring electrodes connected to each spine.

11. A distal tip of a medical probe, the distal tip comprising a spine framework having a proximal end, an intermediate section, and a distal end and extending along a longitudinal axis, the spine framework comprising:
a neck extending from the proximal end;
a plurality of spines extending along the intermediate section, each spine comprising:
a first straight section connected to the neck; and
a second straight section comprising a first end and a second end, the first straight section being connected to the second straight section at a joint intermediate the first end and the second end;
a crown connected to the second straight section and extending to the distal end,
the spine framework being moveable between:
a collapsed configuration, where each connected first straight section and second straight section are oriented at a first angle relative to one another; and
an expanded configuration, where each connected first straight section and second straight section are oriented at a second angle relative to one another, the second angle being smaller than the first angle.

12. The distal tip of claim 11, further comprising a flexible membrane connected to the second straight sections and/or further comprising i) one or more electrodes connected to the flexible membrane and/or ii) the flexible membrane comprising a conical shape in the expanded configuration of the spine framework.

13. The distal tip of claim 11 or claim 12, each spine extending along the longitudinal axis in the collapsed configuration.

14. The distal tip of any of claims 11 to 13, the spine framework defining a lumen therethrough.

15. A system comprising:
a medical probe comprising:
an elongated shaft extending along a longitudinal axis; and
distal tip of a medical probe, the distal tip comprising a spine framework having a proximal end, an intermediate section, and a distal end and extending along a longitudinal axis, the spine framework comprising:
a neck extending from the proximal end; and
a plurality of spines connected to the neck and extending along the intermediate section to the distal end, the spines being movable between:
a collapsed configuration, where the spines extend along the longitudinal axis; and
an expanded configuration, where each spine comprises a curved section that bends away from the longitudinal axis and a planar section extending from the curved section to the distal end, each planar section extending along a plane that is approximately orthogonal to the longitudinal axis;
a guidewire extending through the elongated shaft and the distal tip; and
a guiding sheath that is slidable relative to the distal tip to cause the spines to move between the collapsed configuration and the expanded configuration.
